# EUROPÄISCHE PATENTSCHRIFT

(11) **EP 4 117 579 B1**
(45) Veröffentlichungstag und Bekanntmachung des Hinweises auf die Patenterteilung: **04.11.2026**
(21) Anmeldenummer: 21711811.6
(22) Anmeldetag: 09.03.2021
(51) Int. Cl.: A61F 2/16

(54) **KÜNSTLICHE AUGENLINSE**
ARTIFICIAL EYE LENS
CRISTALLIN OCULAIRE ARTIFICIEL

(30) Priorität: 11.03.2020 DE 102020203121
(43) Veröffentlichungstag der Anmeldung: 18.01.2023
(73) Patentinhaber: Carl Zeiss Meditec AG, 07745 Jena (DE)
(72) Erfinder: GERLACH, Mario, 10589 Berlin (DE)
(74) Vertreter: Nieten, Christoph
(86) Internationale Anmeldenummer: PCT/EP2021/055876
(87) Internationale Veröffentlichungsnummer: WO 2021/180695

(56) Entgegenhaltungen:
- WO-A1-2018/229766
- US-A- 5 203 788
- US-A- 5 984 962
- US-A1- 2014 336 757
- US-B1- 6 730 123

## Beschreibung

Die vorliegende Erfindung betrifft eine Augenlinse mit einem Haptikelement und einem Optikelement, wobei das Optikelement mindestens zwei optische Flächen aufweist und relativ zum Haptikelement unter Überwindung einer Haftreibung bewegt werden kann. Die Erfindung betrifft weiterhin eine Planungseinheit zur Erzeugung von Steuerdaten für eine Korrekturvorrichtung zur Korrektur eines Refraktionsfehlers eines Auges, das eine erfindungsgemäße Augenlinse aufweist, wobei die Korrekturvorrichtung eine Energieeinrichtung zur Bereitstellung einer Energie, eine Fokussiereinrichtung zum Fokussieren der Energie in einem Energiefokus, und eine Steuereinheit zu Steuerung der Korrekturvorrichtung mittels der Steuerdaten umfasst, wobei die Planungseinheit eine Schnittstelle zum Abführen der Steuerdaten an die Steuereinheit aufweist. Weiterhin betrifft die Erfindung eine entsprechende Korrekturvorrichtung. Schließlich betrifft die Erfindung ein Planungsverfahren zur Erzeugung von Steuerdaten für eine Korrekturvorrichtung sowie ein Verfahren zur Korrektur eines Refraktionsfehlers eines Auges.

Bei Intraokularlinsen handelt es sich um künstliche Augenlinsen, die aus einem transparenten Material gefertigt sind. Sie können im Auge anstelle oder zusätzlich zur natürlichen Augenlinse implantiert werden, um beispielsweise die Brechkraft eines myopen, hyperopen oder astigmatischen Auges zu korrigieren. Allgemein kann die Sehkraft eines erkrankten Auges verbessert oder wiederhergestellt werden, beispielsweise bei einer Katarakt-Erkrankung. Dabei sei unter dem Begriff Sehkraft die Qualität einer mit einem Auge wahrgenommenen Szene zu verstehen, die beispielsweise durch Linsentrübung, Astigmatismus, Dezentrierung der optisch wirksamen Flächen aber auch durch nicht angepasste Brechkräfte beeinträchtigt sein kann.

Vor der Implantation einer Intraokularlinse wird das Auge typischerweise vermessen, um in Abhängigkeit beispielsweise der Augenlänge, der Krümmung der Kornea und der geplanten Position der künstlichen Augenlinse im Kapselsack deren benötigte Brechkraft zu ermitteln. Auf diese Weise lässt sich für das optische Gesamtsystem des Auges, das neben der künstlichen Augenlinse beispielsweise auch die Kornea umfasst, eine erwünschte Gesamtbrechkraft erzielen. Da es sich beim Auge jedoch um organisches Gewebe handelt, dessen (mechanische) Eigenschaften sich durch den Eingriff bei einer Implantation oder bei der anschließenden Wundheilung verändern können, kommt es vor, dass die Position der implantierten Augenlinse verschoben oder verdreht ist, und/oder dass die Brechkraft der Augenlinse nicht zu der erwünschten, berechneten Verbesserung der Sehkraft des Auges führen.

Aus diesem Grunde sind verschiedene Vorrichtungen und Verfahren entwickelt worden, die post-operativ eine Änderung der Brechkraft, Position oder Achslage einer implantierten Augenlinse ermöglichen, um so die Sehkraft des Auges zu verbessern.

In DE 101 05 080 B4 ist eine Augenlinse mit einer Haptik vorgeschlagen, die eine Justiervorrichtung aufweist, mit deren Hilfe die Linse unter Verwendung eines Werkzeugs nach der Implantation (und Wundheilung) axial verschoben werden kann. Dazu ist ein erneuter, invasiver chirurgischer Eingriff mit den dabei einhergehenden gesundheitlichen Risiken erforderlich. Weiterhin wird für diesen Eingriff ein Operationssaal (OP) benötigt und ist somit mit erheblichem Kostenaufwand verbunden.

Weiterhin sind Lösungen entwickelt worden, die keinen erneuten chirurgischen Eingriff erfordern: In US 5 984 962 A werden Augenlinsen beschrieben, bei denen die Optik mittels Laserstrahlung oder einem Mikromotor rotiert werden kann. In US 5 203 788 A wird eine eine intraokulare Linse mit Mikromotoren beschrieben, bei der die Brechkraft durch mechanisch bewirkte Verformung der Optik infolge externer Energiezufuhr verändert wird.

In WO 2018229766 A1 ist eine Augenlinse beschrieben, die einen Aktuator aufweist, über den die Linse axial, in ihrer Orientierung oder ihrer Form verändert werden kann. Der Aktuator wird über eine externe Energiequelle betrieben, die beispielsweise einen Laser aufweist. Weiterhin wird in WO 03/057022 A1 eine Augenlinse beschrieben, deren optische Eigenschaften nach der Implantation durch einen externen Stimulus geändert werden können. Dabei umfasst die Linse lichtempfindliche Polymere, die Licht oder Wärme aufnehmen und dadurch ihren Brechungsindex oder die Krümmung der Linse ändern. Ist der gewünschte Zustand erreicht, kann dieser beispielsweise durch Bestrahlung mit dem Licht einer Quecksilberdampflampe "eingefroren" werden; man spricht von einem "Lock-In". Aufgrund der Lichtempfindlichkeit des Linsenmaterials - auch gegenüber Tageslicht - muss der Patient bis nach der Wundheilung, einer Stabilisierung des Implantats im Kapselsack und dem Lock-In für mehrere Wochen eine UV-Schutzbrille tragen, um zu verhindern, dass sich die Eigenschaften der implantierten Augenlinse ungewollt verändern. Nach dem Lock-In ist keine weitere Korrektur der Linse mehr möglich. Zusätzlich erfordert eine Korrektur der vorgeschlagenen Augenlinse ein spezielles Therapieplanungs- und Bestrahlungsgerät und ist somit mit zusätzlichen Kosten für den Arzt verbunden.

In WO 2014/077983 werden eine Augenlinse und einer Vorrichtung beschrieben, die es ermöglichen, den Brechungsindex der Linse durch Bestrahlung mit einem gepulsten Laser mit einer Pulsdauer im Bereich von Femtosekunden (fs-Laser) zu verändern. Dabei wird hydrophiles Linsenmaterial mit Laserpulsen unterhalb der Zerstörschwelle beaufschlagt, um lokal den Wassergehalt zu verändern. Das Verfahren ist zum einen auf hydrophile Augenlinsen beschränkt, und benötigt zum anderen eine spezielle Vorrichtung, die aufgrund des erforderlichen fs-Lasers und des Einsatzes in einem OP sehr kostspielig ist.

In US 2014/0200666 A1 wird eine Augenlinse mit einer Haptik vorgestellt, bei der eine torische Linse mit der Haptik drehbar gekoppelt ist. Dabei wird die Linse gegenüber der Haptik mittels unter Zugkraft stehenden Befestigungen gehalten. Nach der Implantation (und der Wundheilung) lassen sich gezielt einige Befestigungen mit Hilfe eines Lasers durchtrennen, so dass die Linse gegenüber der Haptik rotiert, um beispielweise einen Astigmatismus zu korrigieren. Nachteilig an der beschriebenen Lösung ist, dass die Korrektur nur in sehr wenigen, diskreten Schritten erfolgen kann und nur eingeschränkt reversibel ist.

In US 6 730 123 B1 ist eine Intraokularlinse vorgestellt, die einen optische Kammer aufweist, die unter dem Einfluss einer Flüssigkeit deformierbar ist. Die optische Kammer ist mit einem Reservoir verbunden. Über ein Ventil und eine Pumpe kann das Flüssigkeitsvolumen in der optischen Kammer angepasst werden und somit die Fokussierung der Intraokularlinse.

In US 2014/336757 A1 ist eine akkommodierende Augenlinse beschrieben.

Aufgabe der vorliegenden Erfindung ist es deshalb, eine Augenlinse zu beschreiben, die eine Korrektur der Sehkraft nach der Implantation erlaubt und dabei die diskutierten Nachteile nicht aufweist. Weiterhin ist es Aufgabe der vorliegenden Erfindung, eine Vorrichtung zu beschreiben, die eine post-operative Korrektur der Sehkraft ohne die genannten Nachteile ermöglicht. Erfindungsgemäß wird die Aufgabe durch die Merkmale der unabhängigen Ansprüche gelöst. Bevorzugte Weiterbildungen und Ausgestaltungen sind Gegenstand der abhängigen Ansprüche.

Ein erster Aspekt der Erfindung betrifft eine Augenlinse, die ein Haptikelement und ein Optikelement aufweist. Das Haptikelement ist dazu ausgebildet, im Auge fixiert zu werden, vorzugsweise im Kapselsack oder am Sulcus Ciliaris. Nach einer Implantation der Augenlinse ins Auge und der Wundheilung wächst das Haptikelement ein und steht fest. Es kann beispielsweise als Plattenhaptik oder C-Loop Haptik ausgeformt sein. Das Optikelement weist mindestens zwei optische Flächen (auch "optisch wirksame Flächen" genannt) auf. Diese können refraktiv oder diffraktiv gestaltet sein und auf einer beliebigen Grundform (z.B. sphärisch, asphärisch, torisch, Fresnel-Struktur oder Freiformfläche) aufgebracht sein. Die Augenlinse ist dabei so ausgebildet, dass das Optikelement relativ zum Haptikelement unter Überwindung einer Haftreibung bewegt werden kann. Bei der relativen Bewegung kann es sich um eine laterale Verschiebung in eine oder mehrere Raumrichtungen, eine axiale Verschiebung in eine Raumrichtung oder um eine Drehung um eine Achse handeln. Über die relative Bewegung zwischen Optikelement und Haptikelement wird eine relative Lageänderung zwischen dem Optikelement und den weiteren optisch wirksamen Strukturen im Auge (wie der Kornea) erzielt und somit eine Veränderung der Sehkraft (beispielsweise der Gesamtbrechkraft) des implantierten Auges ermöglicht.

Erfindungsgemäß zeichnet sich die Augenlinse dadurch aus, dass sie weiterhin ein Kompartiment umfasst, das einen Ausgangskanal aufweist und dazu ausgebildet ist, Flüssigkeit aufzunehmen. Die Flüssigkeit kann während oder nach der Implantation durch den Ausgangskanal in das Kompartiment einströmen; es kann sich auch bereits zum Zeitpunkt der Implantation Flüssigkeit im Kompartiment befinden, das über den Ausgangskanal in das Kompartiment gelangt ist.

Weiterhin ist die Augenlinse dazu ausgebildet, einen Energieeintrag in die ins Kompartiment aufgenommenen Flüssigkeit zu ermöglichen. Dazu kann die Transmission für die Energie des Energieeintrags in das Kompartiment mindestens 50% betragen, bevorzugt mindestens 90%, insbesondere bevorzugt mindestens 95%.

Ein Teil der Energie des Energieeintrags kann von der ins Kompartiment aufgenommenen Flüssigkeit absorbiert werden. Die auftretende lokale Intensität kann ein sehr heißes Plasma von mehr als 10000K erzeugen und somit eine schnell expandierende Gasblase aus Flüssigkeitsdampf. Innerhalb der Gasblase kann der Druck beispielsweise auf mehr als 20bar ansteigen. Die Gasblase kann durch ihre Raumforderung und den damit einhergehenden Druckanstieg die umgebende Flüssigkeit verdrängen und einerseits zur Expansion des umgebenden Kompartiments führen (im Rahmen von dessen Elastizität). Andererseits kann zur Schaffung eines Druckausgleichs ein Ausströmen bzw. ein Ausstoß eines Teils der Flüssigkeit durch den Ausgangskanal aus dem Kompartiment erfolgen. Dieser Ausstoß bewirkt einen Rückstoß, dessen Kraftvektor in entgegengesetzter Richtung zum Ausströmen wirkt. Erfindungsgemäß ist der Ausgangskanal des Kompartiments so ausgebildet, dass beim Energieeintrag in die ins Kompartiment aufgenommene Flüssigkeit ein Ausströmen eines Teils der Flüssigkeit durch den Ausgangskanal aus dem Kompartiment derart erfolgt, dass eine Bewegung des Optikelements gegenüber dem Haptikelement unter Überwindung der Haftreibung erzeugt wird. Auf diese Weise lässt sich eine Relativbewegung zwischen den beiden Elementen (Haptikelement und Optikelement) um einen bestimmten Betrag entgegen der Ausstoßrichtung des Flüssigkeitsstroms erzeugen, bis diese durch Gleitreibung und/oder einen Anschlagmechanismus zum Stillstand gebracht wird. Die Haftreibung kann beispielsweise über einen Anpressdruck und die Größe einer Anpressfläche zwischen Haptikelement und Optikelement eingestellt werden. Zur Bereitstellung der erforderlichen Kraft, die Haftreibung zu überwinden, ist das Kompartiment kraftschlüssig mit dem Optikelement oder dem Haptikelement verbunden. Das Kompartiment weist ein räumlich begrenztes Volumen für die Flüssigkeit auf. Der Ausgangskanal ist gerichtet und in entgegengesetzter Richtung zur gewünschten Bewegungsrichtung angeordnet.

Nach der Expansion kann die Gasblase wieder kollabieren und die Raumforderung wieder freigeben. Das Kompartiment nimmt wieder seine ursprüngliche Größe an. Durch die wiedereinströmende Flüssigkeit kehrt sich der Kraftvektor um und das Kompartiment möchte sich in Richtung der Öffnung des Ausgangskanals bewegen. Der Prozess des Kollabierens und Wiedereinströmens ist jedoch langsamer als der der Expansion. Der Kraftvektor ist geringer. Damit sich die Effekte aus gewünschter Bewegung (bei Expansion) und Gegenbewegung (beim Wiedereinströmen) nicht aufheben, ist die Augenlinse entsprechend der Rückstoßdynamik und Reibung bevorzugt so ausgelegt, dass nur bei der Expansion die Haftreibung überwunden wird.

Die erfindungsgemäße Augenlinse erlaubt eine post-operative, nicht-invasive Änderung der Brechkraft in einem implantierten Auge. Dabei kann eine Anpassung zu einem beliebigen Zeitpunkt nach der Implantation erfolgen. Weiterhin kann durch die Wahl der Energiehöhe des Energieeintrags und die Anzahl der Energieeinträge eine Änderung der Brechkraft in beliebig vielen und kleinen (quasi-kontinuierlichen) Korrekturschritten erfolgen. Ein Lock-In ist nicht erforderlich, so dass eine Korrektur beliebig lange nach einer Implantation erfolgen kann.

Die Anpassung der Brechkraft erfolgt mittels einer Bewegung des Optikelements als Ganzes gegenüber dem Haptikelement als Ganzes unter Überwindung einer Haftreibung. Die Bewegung erfolgt unter Beibehaltung der Form der mindestens zwei optischen Flächen.

In einer vorteilhaften Ausgestaltung ist die Augenlinse so ausgestaltet, dass der Energieeintrag in die Flüssigkeit über einen Laserpuls ermöglicht wird. Das raumfordernde Element wird durch eine laserinduzierte Kavitationsblase innerhalb des Kompartiments geschaffen. Dazu sind die Bereiche des Optikelements und/oder des Haptikelements sowie die Bereiche des Kompartiments, die vom Laserpuls zur Erzeugung des Energieeintrags durchstrahlt werden können, so ausgestaltet, dass sie eine hohe Transmission aufweisen. Die Transmission kann für die Wellenlängen des Laserpulses mindestens 50% betragen, vorzugsweise mindestens 90%, insbesondere bevorzugt mindestens 95%.

Gemäß einer besonders vorteilhaften Ausgestaltung der Augenlinse sind Kompartiment und Ausgangskanal so ausgestaltet, dass ein Laserpuls mit einer Pulsenergie zwischen 500µJ und 5mJ die relative Bewegung des Optikelements gegenüber dem Haptikelement erzeugt.

Der Laserimpuls kann beispielsweise von einem Nd:YAG Laser erzeugt werden, dessen Laserstrahl ins flüssigkeitsgefüllten Kompartiment fokussiert wird. Dieser Laserimpuls transportiert innerhalb einer Pulsdauer von wenigen Nanosekunden die erforderliche Energie für den Energieeintrag. Handelsübliche Nd:YAG-Laser kommen beispielsweise bei einer Kapsulotomie zum Einsatz. Die Spezifikationen eines solchen Lasers eignen sich hervorragend für eine Anwendung mit der erfindungsgemäßen Augenlinse. Solche Laser sind in sehr großer Zahl in der klinischen Praxis vorhanden und einfach in der Anwendung. Für eine Korrektur der Brechkraft mit der erfindungsgemäßen Augenlinse sind somit keine kostspieligen Anschaffungen erforderlich. Weiterhin sind keine speziellen Materialmodifikationen erforderlich, vielmehr können für die Augenlinse handelsübliche Materialien verwendet werden, da sie die erforderlichen Transmissionseigenschaften aufweisen.

In einer vorteilhaften Ausgestaltung der Augenlinse handelt es sich bei der Flüssigkeit um eine physiologische Salzlösung (auch "balanced salt solution" oder BSS genannt) oder um Kammerwasser.

Gemäß einer weiteren vorteilhaften Ausgestaltung weist die Augenlinse weiterhin eine Führungsvorrichtung auf, entlang derer das Optikelement gegenüber dem Haptikelement unter Überwindung der Haftreibung bewegt werden kann. Die Führungsvorrichtung kann mit dem Haptikelement oder dem Optikelement verbunden sein. Die Führungsvorrichtung kann auch mehrteilig ausgestaltet sein, wobei ein Führungsvorrichtungsteil mit dem Haptikelement verbunden ist und ein anderer Führungsvorrichtungsteil mit dem Optikelement. Ein Führungsvorrichtungsteil kann auch Teil des Haptikelements oder Optikelement sein. Die Führungsvorrichtung kann beispielsweise als Schiene, Nut oder Gewinde ausgeführt sein.

Die Führungsvorrichtung erlaubt es, die Richtung, in der eine relative Bewegung zwischen Haptikelement und Optikelement erfolgen soll, zusätzlich zur Vorgabe durch die Ausrichtung des Ausgangskanals festzulegen. Dies ist insbesondere bei Drehbewegungen vorteilhaft.

In einer weiteren vorteilhaften Ausgestaltung der Augenlinse weist das Optikelement eine optische Achse auf, und der Ausgangskanal ist dazu ausgebildet, die Bewegung des Optikelements gegenüber dem Haptikelement senkrecht zur optischen Achse, parallel zur optischen Achse und/oder rotierend um die optische Achse zu ermöglichen.

Die optische Achse kann beispielsweise über die Verbindung der Linsenscheitel von zwei optischen Flächen des Optikelements definiert sein. Vorteilhaft sind Haptikelement und Optikelement so gestaltet, dass die optische Achse des Optikelements im implantierten Zustand parallel zur optischen Achse des Auges ist.

Eine Relativbewegung von Optikelement gegenüber Haptikelement senkrecht zur optischen Achse des Optikelements erlaubt es vorteilhaft, die beiden optischen Achsen übereinander zu schieben. Auf diese Weise lassen sich Aberrationen aufgrund von Dezentrier-Fehlern korrigieren und somit die Sehkraft verbessern.

Eine Relativbewegung von Optikelement gegenüber Haptikelement parallel zur optischen Achse des Optikelements erlaubt es vorteilhaft, Abstände zwischen optisch wirksamen Flächen wie der Kornea und den Flächen des Optikelements anzupassen. Auf diese Weise lässt sich über die resultierende Brechkraftänderung des gesamten Auges die Sehkraft verbessern.

Eine rotatorische Relativbewegung von Optikelement gegenüber Haptikelement um die optische Achse des Optikelements erlaubt es vorteilhaft, die Achslage eines torischen Optikelements zu verändern und somit den Astigmatismus des implantierten Auges zu korrigieren.

Es ist besonders vorteilhaft, wenn die genannten Bewegungsrichtungen über eine Führungsvorrichtung präzise vorgegeben sind; dies gilt insbesondere für eine Rotation um die optische Achse des Optikelements.

Gemäß einer besonders vorteilhaften Ausgestaltung weist das Haptikelement einen Optikbereich auf, der mindestens zwei optisch wirksame Flächen umfasst. Diese Flächen des Optikbereichs des Haptikelement können im implantierten Zustand gemeinsame mit den optischen Flächen des Optikelements zur abbildenden Lichtführung auf eine Retina des Auges genutzt werden. Die optischen Flächen des Optikbereichs des Haptikelements (fortan auch als "optische Flächen des Haptikelements" bezeichnet) können refraktiv oder diffraktiv gestaltet sein und auf einer beliebigen Grundform (z.B. sphärisch, asphärisch, torisch, Fresnel-Struktur oder Freiformfläche) aufgebracht sein. Der Optikbereich ist mit dem Haptikelement fest verbunden. Die Augenlinse weist somit mindestens vier optisch wirksame Flächen auf.

Vorteilhaft ist eine optische Achse des Optikbereichs des Haptikelements zur optischen Achse des Optikelements parallel. Eine Relativbewegung von Optikelement gegenüber Haptikelement erlaubt es dann, die beiden optischen Achsen übereinander zu setzen. Auf diese Weise lassen sich Aberrationen aufgrund von Dezentrier-Fehlern korrigieren und somit die Sehkraft verbessern.

Durch die höhere Anzahl von optisch wirksamen Flächen erhöht sich die Anzahl von Parametern wie Linsenkrümmung, Brechzahl oder Abbe-Zahl, die für eine Verbesserung der Sehkraft eines implantierten Auges zu Verfügung stehen. Sie erlaubt zudem eine weitere Verbesserung der Korrektur von verbleibenden, chromatischen Fehlern.

Von den optisch wirksamen Flächen des Haptikelements und des Optikelements kann eine Fläche eine torische Form aufweisen. Vorteilhaft weist eine optisch wirksame Fläche des Optikelements eine torische Form auf. Zusätzlich kann eine optisch wirksame Fläche des Optikbereichs des Haptikelements eine torische Form aufweisen. Eine rotatorische Relativbewegung des Optikelements gegenüber Haptikelement erlaubt es dann vorteilhaft, die Achslage des torischen Optikelements zu verändern (ggf. gegenüber der Achslage des torischen Optikbereichs des Haptikelements) und somit eine Abstimmung der Achslage für astigmatische Korrekturen vorzunehmen. Auf diese Weise lassen sich achslagebedingte astigmatische Abbildungsfehler korrigieren und somit die Sehkraft verbessern. Vorteilhaft sind dabei die optischen Achsen von Optikelement und Optikbereich des Haptikelements identisch.

Es kann auch von den optisch wirksamen Flächen des Haptikelements und des Optikelements jeweils mindestens eine als Freiformflächen dritter Ordnung nach Lohmann oder Alvarez ausgestaltet sein. Das Design derartiger Flächen ist hinreichend in der Fachliteratur beschrieben und nicht Gegenstand der Erfindung. Eine laterale oder rotatorische, relative Verschiebung von Haptikelement und Optikelement erlaubt vorteilhaft eine Anpassung der Brechkraft und kann somit die Sehkraft des implantierten Auges post-operativ verbessern.

In einer weiteren besonders vorteilhaften Ausgestaltung weist die Augenlinse mindestens ein weiteres Kompartiment auf. Dabei ist der Ausgangskanal des weiteren Kompartiments so ausgebildet, dass beim Energieeintrag in die ins weitere Kompartiment aufgenommene Flüssigkeit ein Ausströmen eines Teils der Flüssigkeit durch den Ausgangskanal aus dem weiteren Kompartiment erfolgt. Dabei wird eine Bewegung des Optikelements gegenüber dem Haptikelement unter Überwindung der Haftreibung mit einer Bewegungsrichtung erzeugt, die verschieden ist von der Bewegungsrichtung, die durch das Ausströmen der Flüssigkeit aus dem ersten Kompartiment erzeugt wird. Mit anderen Worten lässt sich mittels Energieeintrag in zwei verschiedene Kompartimente eine relative Bewegung zwischen Haptikelement und Optikelement in zwei unterschiedliche Richtungen erzeugen.

Die beiden Bewegungsrichtungen können beispielsweise entgegengesetzt sein. Auf diese Weise lässt sich eine vorgenommene Korrektur der relativen Lagen von Haptikelement und Optikelement wieder rückgängig machen. Dies erlaubt eine reversible Lagekorrektur für eine Augenlinse. Weist zusätzlich das Haptikelement optisch wirksame Flächen auf (wie beispielsweise Freiformflächen nach Lohman oder Alvarez), so lässt sich die Brechkraft umkehrbar anpassen. Im Falle einer rotatorischen Bewegung einer torischen Optik kann der maximal erforderliche Verstell-Winkel verkleinert werden (von bis zu 180° auf bis zu 90°).

Die Bewegungsrichtungen können auch einen Winkel zwischen 0° und 180° aufweisen, vorzugsweise zwischen 80° und 100°, insbesondere vorzugsweise 90°. Auf diese Weise lassen sich beispielsweise beliebige Verschiebungen zwischen der optischen Achse des Optikelementes und des Auges korrigieren oder eine laterale Verschiebung der optischen Achse des Optikelements und zusätzliche eine axiale Verschiebung erzielen.

Die Verwendung von mehr als zwei Kompartimenten in einer Augenlinse ist ebenfalls denkbar und vorteilhaft, um reversible Relativbewegungen in mehrere Richtungen zu erlauben - wie beispielsweise lateral und/oder axial und/oder Rotation. Durch die Verwendung eines Führungselementes lassen sich die Bewegungsrichtungen weiter vorteilhaft präzisieren.

Haptikelemente und Optikelement der Augenlinse können in einer Ausgestaltung so geformt sein, dass das Optikelement nach einer Implantation ins Auge operativ entfernt und gegen ein anderes Optikelement ausgetauscht werden kann. Die dazu erforderliche Bewegung der Elemente gegeneinander kann dabei von den durch Energieeintrag in ein Kompartiment erzeugte Bewegungen verschieden sein. Es kann dafür auch eine Bewegung erforderlich sein, die eine Kraft erfordert, die durch Energieeintrag in ein Kompartiment nicht erzeugt werden kann.

Ein zweiter Aspekt der Erfindung betrifft eine Planungseinheit zur Erzeugung von Steuerdaten für eine Korrekturvorrichtung zur Korrektur eines Refraktionsfehlers eines Auges, das eine Augenlinse nach einer der oben beschriebenen Ausführungen aufweist. Die Korrekturvorrichtung umfasst eine Energieeinrichtung zur Bereitstellung einer Energie. Bei der Energieeinrichtung kann es sich beispielsweise um eine Lichtquelle wie einen Lasereinrichtung handeln. Es kann sich auch um eine Ultraschallquelle handeln. Weiterhin umfasst die Korrekturvorrichtung eine Fokussiereinrichtung zum Fokussieren der Energie in einem Energiefokus. Dabei kann es sich um eine Optik handeln, die beispielsweise aus einer oder mehreren Linsen aufgebaut ist. Es kann sich auch um eine Vorrichtung handeln, die Ultraschall in einem Punkt fokussiert. Weiterhin weist die Korrekturvorrichtung eine Steuereinheit zur Steuerung mittels der Steuerdaten auf. Dazu ist die Steuereinheit so ausgebildet, dass sie Signaldaten, die unter Berücksichtigung der Steuerdaten ermittelt werden, über eine Signaldatenleitungen an die Energieeinheit übertragen kann. Die Steuereinheit kann als Computer ausgestaltet sein. Ferner weist die Planungseinheit bevorzugt eine Schnittstelle auf, über die Steuerdaten der Steuereinheit drahtgebunden oder auch drahtlos zugeführt werden können.

Erfindungsgemäß ist die Planungseinheit dazu ausgebildet ist, weitere Steuerdaten zu erzeugen, mit denen die Korrekturvorrichtung so angesteuert werden kann, dass die Energieeinrichtung die Energie bereitstellt, die im Kompartiment der Augenlinse den Energieeintrag ermöglicht, der die Bewegung des Optikelements gegenüber dem Haptikelement unter Überwindung der Haftreibung erzeugt.

Bei den weiteren Steuerdaten kann es sich beispielsweise um Parameter für die Ansteuerung der Energiequelle handeln, wie etwa die Menge von Energie, die in das Kompartiment eingetragen werden soll, oder die Dauer, über die der Eintrag erfolgen soll, oder eine Anzahl von Energiepulsen. Die Steuerdaten können auf Basis von zuvor ermittelten Informationen über die zu korrigierende Fehlsichtigkeit bestimmt werden. Weiterhin können die Steuerdaten unter Berücksichtigung von Informationen über die Augenlinse ermittelt werden, beispielweise der möglichen Bewegungsrichtungen zwischen Haptikelement und Optikelement (gegeben durch die Anordnung des Ausgangskanals des Kompartiments und/oder durch eine Führungsvorrichtung). Außerdem kann die Haftreibung zwischen Haptikelement und Optikelement berücksichtigt werden. Zusätzlich können Informationen über den Zusammenhang zwischen einem relativen Versatz von Haptikelement und Optikelement und einer Änderung der optischen Eigenschaften (wie Brechkraft oder Achslage eines Torus) in die Berechnungen einfließen.

Die Planungseinheit kann als Computer ausgestaltet sein, der einen Prozessor und einen Speicher aufweist. Die Planungseinheit kann auch Teil eines Computers sein, der zusätzlich beispielsweise die Steuereinheit umfasst.

Die Korrekturvorrichtung ist vorteilhaft dazu ausgebildet, die Lage des Energiefokus in der Augenlinse zu verschieben. Dies kann dadurch gelöst sein, dass die (in sich starre) Einheit aus Energieeinrichtung und Fokussiereinrichtung frei beweglich vor dem Auge positioniert werden kann; dazu kann die Einheit als handgehaltener Applikator ausgebildet sein. Alternativ kann die Korrekturvorrichtung eine Kinn- und/oder Stirnstütze oder ein Kontaktglas aufweisen, um das Auge gegenüber der Korrekturvorrichtung zu positionieren. Die Einheit ist dann innerhalb der Korrekturvorrichtung beweglich ausgeführt, um den Energiefokus in der Augenlinse zu verschieben. Alternativ kann die Korrekturvorrichtung auch eine Ablenkeinrichtung aufweisen, die entlang eines Energiestrahls zwischen der Energieeinrichtung und der Fokussiervorrichtung angeordnet ist. Diese erlaubt ein Verschieben des Energiefokus in der Augenlinse des Auges. Bei der Verschiebeeinrichtung kann es sich um eine optische Ablenkeinrichtung handeln wie beispielsweise einen Scanner. Es kann sich auch um eine Einrichtung handeln, die eine Ultraschallfokus ablenken kann. Ablenkeinrichtung und ggf. auch die Fokussiereinrichtung erlaube es, den Energiefokus dreidimensional im Auge zu verschieben. Die Planungseinheit kann auch Steuerdaten erzeugen, die von der Steuereinheit in Signaldaten für die Fokussiereinrichtung und/oder die Ablenkeinrichtung umgewandelt werden und die über entsprechende Signaldatenleitungen weitergeleitet werden.

Die erfindungsgemäße Planungseinheit ermöglicht somit, Steuerdaten für eine Korrekturvorrichtung zu ermitteln, die bei ihrer Ausführung in einer post-operative Korrektur mittels der Korrekturvorrichtung die Sehkraft eines implantierten Auges verbessern kann.

Es sei darauf hingewiesen, dass die erfindungsgemäße Planungseinheit die Steuerdaten erzeugen kann, ohne dass das Auge mit der Korrekturvorrichtung verbunden ist. Vielmehr können die Steuerdaten zeitlich getrennt und vor der eigentlichen Durchführung der Korrektur erzeugt werden.

Die Erfindung betrifft weiterhin eine Korrekturvorrichtung zur Korrektur eines Refraktionsfehlers eines Auges, das eine Augenlinse nach einer der oben beschriebenen Ausführungen aufweist. Die Korrekturvorrichtung weist eine Energieeinrichtung zur Bereitstellung einer Energie, eine Fokussiereinrichtung zum Fokussieren der Energie in einem Energiefokus und eine Steuereinheit zu Steuerung der Korrekturvorrichtung mittels Steuerdaten auf. Zusätzlich weist die Korrekturvorrichtung eine Planungseinheit zur Generierung der Steuerdaten auf. Die Planungseinheit ist dabei so ausgebildet, dass die die Steuerdaten derart generiert, wie es in den oben beschriebenen Ausführungsformen ausgeführt ist.

Die Erfindung betrifft weiterhin ein Planungsverfahren zur Erzeugung von Steuerdaten für eine Korrekturvorrichtung zur Korrektur eines Refraktionsfehlers eines Auges, das eine Augenlinse nach einer der oben beschriebenen Ausführungen aufweist. Dabei weist die Korrekturvorrichtung eine Energieeinrichtung zur Bereitstellung einer Energie, eine Fokussiereinrichtung zum Fokussieren der Energie in einem Energiefokus und eine Steuereinheit zu Steuerung der Korrekturvorrichtung mittels der Steuerdaten auf.

Das erfindungsgemäß Planungsverfahren weist den Verfahrensschritt einer Erzeugung weiterer Steuerdaten auf, mit denen die Korrekturvorrichtung so angesteuert werden kann, dass die Energieeinrichtung die Energie bereitstellt, der im Kompartiment der Augenlinse den Energieeintrag ermöglicht, der die Bewegung des Optikelements gegenüber dem Haptikelement unter Überwindung der Haftreibung erzeugt. Weiterhin umfasst das Planungsverfahren den Verfahrensschritt einer Zuführung der Steuerdaten zu der Steuereinheit der Korrekturvorrichtung.

Die Erfindung betrifft weiterhin ein Verfahren zur Korrektur eines Refraktionsfehlers eines Auges, das eine Augenlinse nach einer der oben beschriebenen Ausführungen aufweist, mit Hilfe einer Korrekturvorrichtung. Dabei weist die Korrekturvorrichtung eine Energieeinrichtung zur Bereitstellung einer Energie, eine Fokussiereinrichtung zum Fokussieren der Energie in einem Energiefokus und eine Steuereinheit zu Steuerung der Korrekturvorrichtung mittels Steuerdaten auf.

Erfindungsgemäß umfasst das Verfahren einen Schritt zur Erzeugung weiterer Steuerdaten, mit denen die Korrekturvorrichtung so angesteuert werden kann, dass die Energieeinrichtung eine Energie bereitstellt, der im Kompartiment der Augenlinse den Energieeintrag ermöglicht, der die Bewegung des Optikelements gegenüber dem Haptikelement unter Überwindung der Haftreibung erzeugt. Weiterhin umfasst das Verfahren ein Zuführen der Steuerdaten an die Steuereinheit der Korrekturvorrichtung. Darüber hinaus weist das Verfahren den Schritt auf, einen Eintrag von Energie gemäß der Steuerdaten ins Kompartiment der Augenlinse zur Erzeugung einer Bewegung des Optikelements gegenüber dem Haptikelement unter Überwindung der Haftreibung hervorzurufen. Die Steuerdaten werden dazu in der Steuereinheit in Signaldaten umgewandelt und an die Energieeinrichtung übertragen.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, näher erläutert. Es zeigen:
Fig. 1a eine perspektivische Darstellung eines ersten Ausführungsbeispiels einer erfindungsgemäßen Augenlinse;
Fig. 1b eine perspektivische Darstellung des Ausführungsbeispiels einer erfindungsgemäßen Augenlinse aus Fig. 1a mit einer alternativen Haptik;
Fig. 2a bis 2d eine schematische Darstellung des Funktionsprinzips der relativen Bewegungen durch Energieeintrag;
Fig. 3 eine Aufsicht eines zweiten Ausführungsbeispiels einer erfindungsgemäßen Augenlinse;
Fig. 4 einen Ausschnitt einer Seitenansicht einer Variante des zweiten Ausführungsbeispiels mit einem Haptikelement, das einen Optikbereich aufweist;
Fig. 5 eine Aufsicht eines dritten Ausführungsbeispiels einer erfindungsgemäßen Augenlinse;
Fig. 6 einen Ausschnitt einer Seitenansicht einer Variante des dritten Ausführungsbeispiels mit einem Haptikelement, das einen Optikbereich aufweist;
Fig. 7 einen Ausschnitt einer Seitenansicht einer weiteren Variante des dritten Ausführungsbeispiels mit einem Haptikelement, das einen Optikbereich aufweist;
Fig. 8 eine Seitenansicht eines vierten Ausführungsbeispiels einer erfindungsgemäßen Augenlinse;
Fig. 9 eine Seitenansicht eines fünften Ausführungsbeispiels einer erfindungsgemäßen Augenlinse;
Fig. 10 eine schematische Darstellung einer Korrekturvorrichtung.

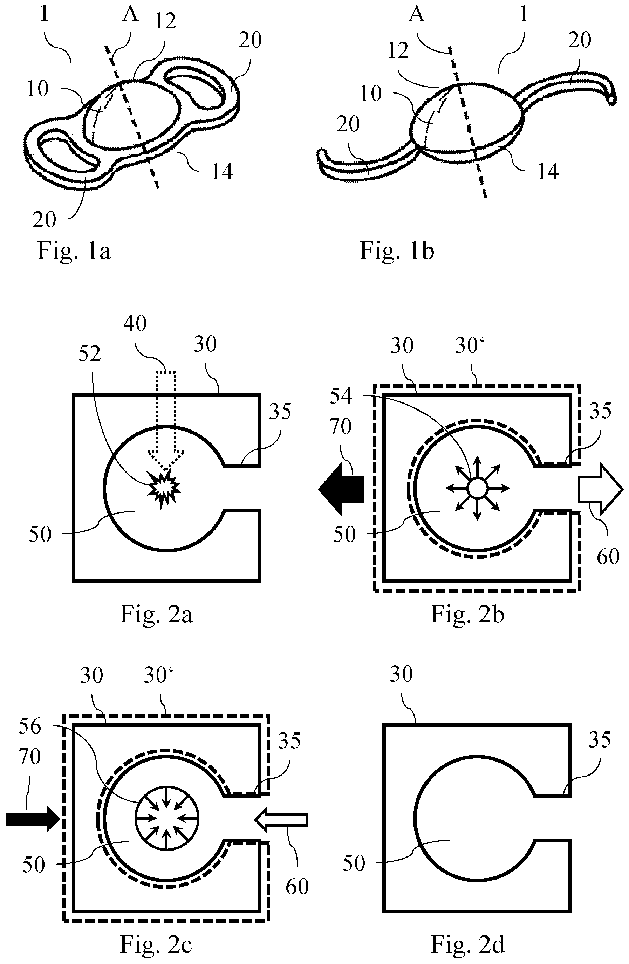

**In** Fig. 1a ist eine perspektivische Darstellung eines ersten Ausführungsbeispiels einer erfindungsgemäßen Augenlinse 1 gezeigt. Die Augenlinse 1 umfasst ein Haptikelement 20 und ein Optikelement 10, das eine im implantierten Zustand der Kornea zugewandte Vorderseite 12 und eine im implantierten Zustand der Retina zugewandte Rückseite 14 aufweist. Mittels der Haptik 20 kann die Augenlinse 1 im Kapselsack des Auges gehalten oder fixiert werden. Die Augenlinse 1 ist über einen kleinen Schnitt in ein Auge einführbar. Die Vorderseite 12 und die Rückseite 14 des Optikelements 10 sind aufgrund ihrer lichtleitenden Eigenschaften im Zusammenspiel mit den weiteren optisch wirksamen Strukturen des Auges wie der Kornea für die optischen Abbildungseigenschaften des Auges, dessen Gesamtbrechkraft und für dessen Sehkraft verantwortlich. Eine optische Achse A ist als gestrichelte Linie als Verbindung der Linsenscheitel der Vorderseite 12 und der Rückseite 14 eingezeichnet.

In Fig. 1b ist eine perspektivische Darstellung eines weiteren Ausführungsbeispiels einer Augenlinse 1 gezeigt. Sie unterscheidet sich von der Ausführung in Fig. 1a darin, dass sie unterschiedliches Haptikelement 20 aufweist.

Grundsätzlich können auch anderweitig geformte und ausgestaltete Haptikelemente 20 vorgesehen sein. Es kann auch ein Haptikelement 20 vorgesehen sein, das ein Fixieren der Augenlinse 1 im Sulcus Ciliaris erlaubt.

In den Figuren 2a bis 2d ist eine schematische Darstellung des Funktionsprinzips der relativen Bewegungen durch Energieeintrag gezeigt. Das in den Figuren gezeigte Kompartiment 30 weist einen Ausgangskanal 35 auf. Im Kompartiment 30 befindet sich eine Flüssigkeit 50 wie beispielsweise Kammerwasser. In Fig. 2a erfolgt mit Hilfe eines Laserpulses 40 (dargestellt als gepunkteter Pfeil) ein Energieeintrag. Dadurch wird in der im Kompartiment 30 enthaltenen Flüssigkeit 50 binnen eines kurzen Zeitraums ein Plasma 52 erzeugt. Dieses erzeugt als raumforderndes Element eine expandierende Gasblase 54 (siehe Fig. 2b, die Expansion wird durch acht auswärts gerichtete Pfeile dargestellt). Die expandierende Gasblase 54 führt einerseits zu einer Expansion des umgebenden Kompartiments (gestrichelt dargestellt mit Bezugszeichen 30'). Andererseits wird ein Ausströmen oder Ausstoß der umgebenden Flüssigkeit 50 durch den Ausgangskanal 35 hervorgerufen. Der resultierende Flüssigkeitsstrom 60 ist als nicht-ausgefüllter Pfeil dargestellt. Der Flüssigkeitsstrom 60 erzeugt eine Kraft, dessen Kraftvektor 70 (schwarz ausgefüllter Pfeil) dem Flüssigkeitsstrom 60 entgegengerichtet ist.

Nach der Expansion kollabiert die Gasblase 56 wieder (siehe Fig. 2c, das Kollabieren wird durch acht einwärts gerichtete Pfeile dargestellt). Dadurch wird die Raumforderung wieder frei. Das expandierte Kompartiment 30' komprimiert sich wieder auf seine ursprüngliche Größe (dargestellt durch das Kompartiment mit Bezugszeichen 30). Gleichzeitig strömt Flüssigkeit 50 durch den Ausgangskanal 35 zurück in das Kompartiment 30. Der Flüssigkeitsstrom 60 und der daraus resultierende Kraftvektor 70 kehren sich in ihrer Richtung um gegenüber dem Zeitpunkt der Expansion der Gasblase. Da die Expansion schneller erfolgt als das Kollabieren, sind die Pfeile für die Flüssigkeitsströme 60 und Kraftvektoren 70 für die Expansion kürzer und breiter dargestellt als für das Kollabieren (vgl. Fig. 2b und Fig. 2c).

Nach dem Kollabieren der Gasblase nimmt das Kompartiment 30 wieder die Größe an, wie sie vor dem Energieeintrag war (vgl. Fig. 2d).

Vorteilhaft ist das Kompartiment 30 kraftschlüssig mit dem Haptikelement 20 oder vorzugsweise mit dem Optikelement 10 verbunden. Dabei sind erfindungsgemäß der Austrittskanal 35 sowie Haptikelement 20 und Optikelement 10 so ausgestaltet, dass der bei einer Expansion auftretende Kraftvektor 70 die Haftreibung zwischen Haptikelement 20 und Optikelement 10 überschreitet. Auf diese Weise wird durch den Energieeintrag eine relative Bewegung zwischen Haptikelement 20 und Optikelement 10 ermöglicht, die dem Flüssigkeitsstrom 60 entgegengerichtet ist.

Vorteilhaft sind weiterhin der Austrittskanal 35 sowie Haptikelement 20 und Optikelement 10 so ausgestaltet, dass der beim Kollabieren auftretende Kraftvektor 70 die Haftreibung zwischen Haptikelement 20 und Optikelement 10 nicht überschreitet. Auf diese Weise wird sichergestellt, dass nach Expansion und Kollabieren der Gasblase eine andere relative Lage von Haptikelement 20 und Optikelement 10 vorliegt als vor dem Energieeintrag.

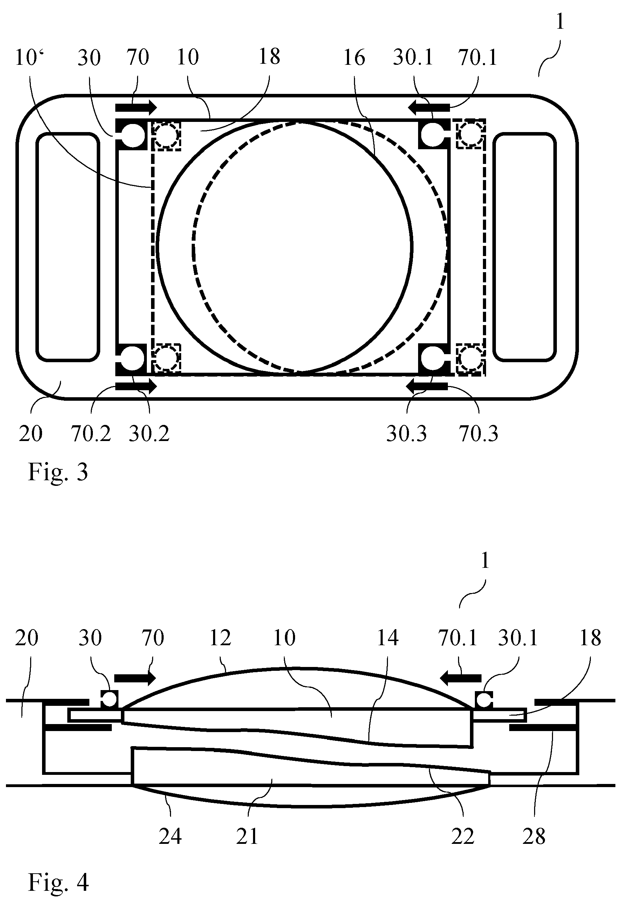

In Fig. 3 ist eine Aufsicht auf eine erfindungsgemäße Augenlinse 1 für ein zweites Ausführungsbeispiel gezeigt. Das Haptikelement 20 ist für eine Implantation in den Kapselsack oder den Sulcus Ciliaris ausgelegt und weist die Form einer Plattenhaptik auf. In diesem Beispiel umfasst das Optikelement 10 neben einer optischen wirksamen Zone 16 (dargestellt als Kreis), die durch die zwei optisch wirksamen Flächen gegeben ist, auch eine Fassungszone 18 auf. Im Ausführungsbeispiel sind vier Kompartimente (30, 30.1, 30.2 und 30.3) kraftschlüssig mit der Fassungszone 18 und somit kraftschlüssig mit dem Optikelement 10 verbunden. Die Ausgangskanäle 35 der Kompartimente (30, 30.1, 30.2 und 30.3) sind dabei so angeordnet, dass bei einem Energieeintrag jeweils zwei Kompartimente eine Bewegung des Optikelements 10 gegenüber dem Haptikelement 20 in dieselbe Richtung erzeugen (30 und 30.2 bzw. 30.1 und 30.3). Die beiden möglichen Bewegungsrichtungen sind gerade entgegengesetzt; die Kraftvektoren 70 und 70.2 zeigen in entgegengesetzte Richtungen zu den Kraftvektoren 70.1 und 70.3. Das nach einem Energieeintrag (beispielsweise in Kompartiment 30 oder 30.2) verschobene Optikelement 10' ist gestrichelt eingezeichnet. Eine Ausführung mit zwei Kompartimenten für dieselbe Bewegungsrichtung kann vorteilhaft sein, wenn die Zugänglichkeit eines Kompartiments für einen Energieeintrag durch eine Verschiebung von Optikelement 10 gegenüber Haptikelement 20 eingeschränkt ist.

Das Ausführungsbeispiel zeigt somit eine Augenlinse 1, die eine laterale Verschiebung des Optikelements 10 erlaubt, um beispielsweise die optische Achse A des Optikelements 10 mit der optischen Achse des Auges in Übereinstimmung zu bringen und somit die Sehkraft des Auges zu verbessern.

In einer in Fig. 4 gezeigten Variante des zweiten Ausführungsbeispiels weist das Haptikelement 20 einen Optikbereich 21 mit zwei optisch wirksame Flächen auf: einer im implantierten Zustand der Kornea zugewandten Vorderseite 22 des Optikbereichs 21 und einer im implantierten Zustand der Retina zugewandten Rückseite 24 des Optikbereichs 21. Dabei ist jeweils mindestens eine der beiden optische wirksamen Flächen von Optikelement 10 und Haptikelement 20 als Freiformfläche dritter Ordnung nach Lohmann oder Alvarez ausgebildet. Im gezeigten Beispiel handelt es sich um die Rückseite 14 des Optikelements 10 und um die Vorderseite 24 des Optikbereichs 21 des Haptikelements 20. Gezeigt ist hier ein Ausschnitt der Augenlinse 1 in einer Seitenansicht. Am Optikelement 10 sind in kraftschlüssiger Verbindung ein Kompartiment 30 und ein Kompartiment 30.1 angebracht. Die Ausgangskanäle und die damit verknüpften Flüssigkeitsströme bei Energieeintrag sind so ausgerichtet, dass die Kraftvektoren 70 und 70.1 einer geeigneten Verschieberichtung entspricht, welche durch das Design der Lohmann- oder Alvarez-Flächen vorgegeben sind; dabei sind die Kraftvektoren 70 und 70.1 einander entgegengerichtet. So ist gewährleistet, dass die Einstellung des Brechwerts umkehrbar in beide Richtungen erfolgen kann. Das

Haptikelement 20 und das Optikelement 10 sind über eine lineare Führungsvorrichtung gegeneinander verschiebbar. Die Führungsvorrichtung ist hier zweiteilig ausgestaltet, wobei ein Führungsvorrichtungsteil 28 mit dem Haptikelement 20 verbunden ist und ein anderer Führungsvorrichtungsteil Teil der Fassungszone 18 des Optikelements 10 ist.

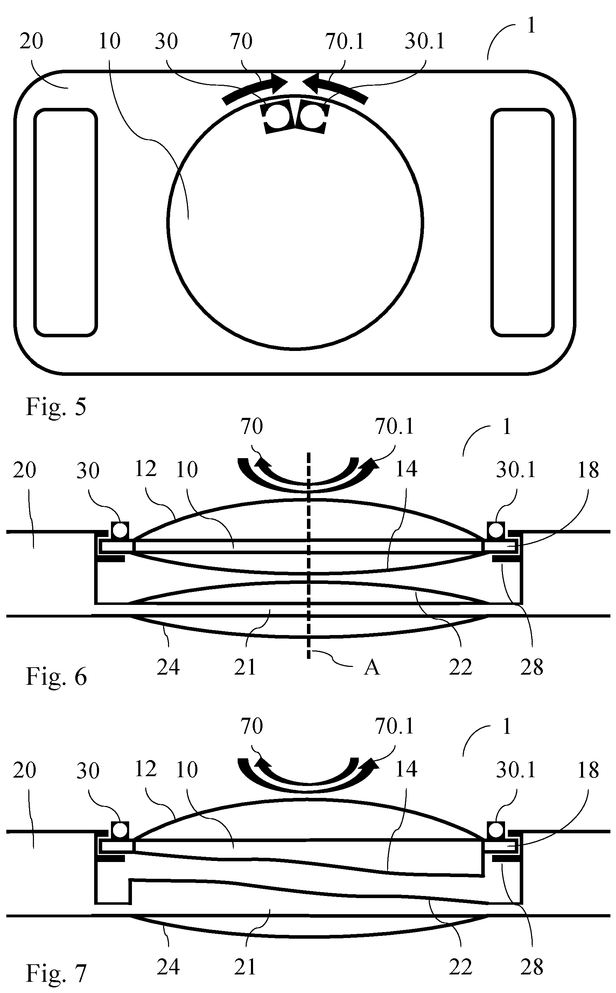

In Fig. 5 ist eine Aufsicht auf eine erfindungsgemäße Augenlinse 1 für ein drittes Ausführungsbeispiel gezeigt. Das Haptikelement 20 ist auch hier für eine Implantation in den Kapselsack oder den Sulcus Ciliaris ausgelegt und weist die Form einer Plattenhaptik auf. Im Ausführungsbeispiel sind zwei Kompartimente (30, 30.1) kraftschlüssig mit dem Optikelement 10 verbunden. Die Ausgangskanäle der Kompartimente sind dabei so angeordnet, dass bei einem Energieeintrag in Kompartiment 30 eine Bewegung des Optikelements 10 gegenüber dem Haptikelement 20 im Uhrzeigersinn erzeugt wird, während ein Energieeintrag in das zweite Kompartiment 30.1 eine Bewegung gegen den Uhrzeigersinn erzeugt. Die Ausgangskanäle 35 sind dazu vorteilhaft tangential zum Rand des Optikelements 10 orientiert. Die beiden möglichen Bewegungsrichtungen sind gerade entgegengesetzt; der Kraftvektor 70 zeigt in entgegengesetzte Richtungen zu Kraftvektor 70.1. Beide sind wegen der rotatorischen Bewegung gekrümmt dargestellt. Die Bewegungsrichtungen können über eine Führungsvorrichtung (nicht eingezeichnet) vorgegeben werden.

Das Ausführungsbeispiel zeigt somit eine Augenlinse 1, die eine Rotation des Optikelements 10 erlaubt, um beispielsweise die Achslage einer torischen Linse zu drehen und somit die Sehkraft des Auges durch eine Korrektur von Astigmatismus zu verbessern. Die Kompartimente (30, 30.1) können sich in einer optisch wirksamen Zone (nicht eingezeichnet) befinden oder außerhalb davon in einer Fassungszone (nicht eingezeichnet).

**In** einer Variante des gezeigten Ausführungsbeispiels weist das Haptikelement 20 einen Optikbereich 21 mit zwei optisch wirksamen Flächen auf: eine im implantierten Zustand der Kornea zugewandte Vorderseite 22 des Optikbereichs 21 und eine im implantierten Zustand der Retina zugewandte Rückseite 24 des Optikbereichs 21. Diese Variante ist in einem Ausschnitt für eine Seitenansicht in Fig. 6 dargestellt. Dabei weist die Vorderseite 12 des Optikelements 10 eine torische Form auf. Auch hier sind die Ausgangskanäle der Kompartimente so angeordnet, dass bei einem Energieeintrag in Kompartiment 30 eine Bewegung des Optikelements 10 gegenüber dem Haptikelement 20 im Uhrzeigersinn erzeugt wird, während ein Energieeintrag in das zweite Kompartiment 30.1 eine Bewegung gegen den Uhrzeigersinn erzeugt (gegenüber dem Haptikelement 20 und dessen Optikbereich 21) wird. Die Ausgangskanäle 35 sind dazu auch hier vorteilhaft tangential zum Rand des Optikelements 10 orientiert. Die beiden möglichen Bewegungsrichtungen sind gerade entgegengesetzt; der Kraftvektor 70 zeigen in entgegengesetzte Richtungen zu Kraftvektor 70.1. Die Führungsvorrichtung ist hier zweiteilig ausgestaltet, wobei ein Führungsvorrichtungsteil 28 mit dem Haptikelement 20 verbunden ist und ein anderer Führungsvorrichtungsteil Teil der Fassungszone 18 des Optikelements 10 ist. Eine rotatorische Relativbewegung des Optikelements 10 gegenüber Haptikelement 20 (bzw. dessen Optikbereich 21) erlaubt es hier vorteilhaft, die Achslage des torischen Optikelements 10 zu verändern und somit eine Abstimmung der Achslage für astigmatische Korrekturen vorzunehmen.

**In** einer weiteren Variante des dritten Ausführungsbeispiels weist das Haptikelement 20 einen Optikbereich 21 mit zwei optisch wirksamen Flächen auf: eine im implantierten Zustand der Kornea zugewandte Vorderseite 22 des Optikbereichs 21 und eine im implantierten Zustand der Retina zugewandte Rückseite 24 des Optikbereichs 21. Diese zweite Variante ist in einem Ausschnitt für eine Seitenansicht in Fig. 7 dargestellt. Hier ist jeweils mindestens eine der beiden optische wirksamen Flächen von Optikelement 10 und Haptikelement 20 als Freiformfläche dritter Ordnung nach Lohmann oder Alvarez ausgebildet. Im gezeigten Beispiel handelt es sich um die Rückseite 14 des Optikelements 10 und um die Vorderseite 24 des Optikbereichs 21 des Haptikelements 20. Am Optikelement 10 ist in kraftschlüssiger Verbindung ein Kompartiment 30 angebracht. Der Ausgangskanal 35 und der damit verknüpfte Flüssigkeitsstrom 60 bei Energieeintrag ist so ausgerichtet, dass der Kraftvektor 70 einer Rotation der optisch wirksamen Flächen (12 und 24 gegen 22 und 24) gegeneinander um eine Achse entspricht, welche durch das Design der Lohmann- oder Alvarez-Flächen vorgegeben ist. Zusätzlich ist ein Kompartiment 30.1 (mit entgegengesetztem Kraftvektor 70.1 bei Energieeintrag) kraftschlüssig mit Optikelement 10 verbunden. So ist gewährleistet, dass die Einstellung des Brechwerts umkehrbar in beide Richtungen erfolgen kann.

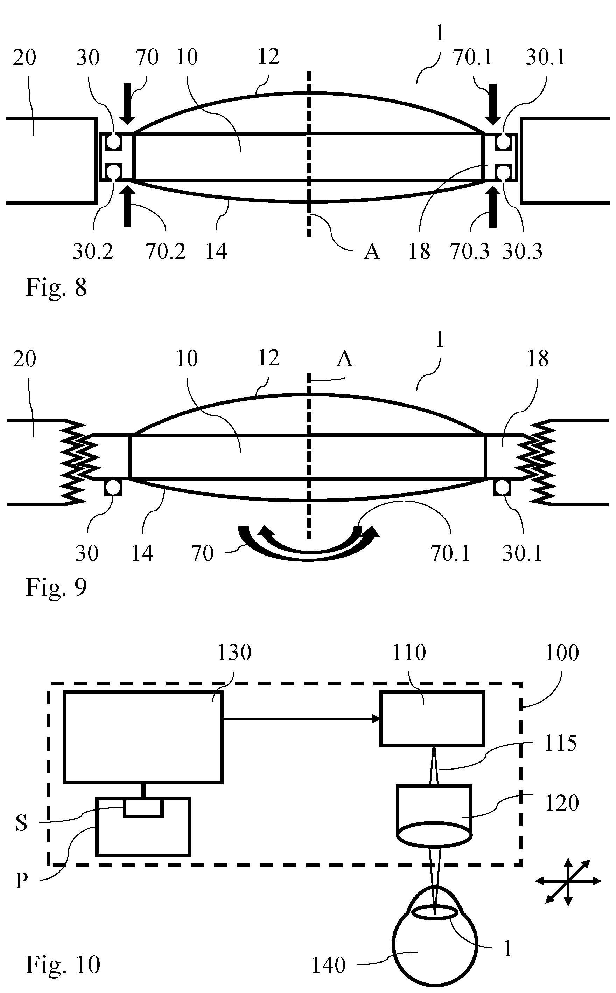

In Fig. 8 ist die Seitenansicht eines vierten Ausführungsbeispiels einer erfindungsgemäßen Augenlinse 1 gezeigt. Das Haptikelement 20 ist nur ausschnittsweise dargestellt. Das Optikelement 10 umfasst neben der Vorderseite 12 und der Rückseite 14 auch eine Fassungszone 18. In dieser Fassungszone 18 sind Kompartimente integriert (und somit kraftschlüssig verbunden), wobei die zwei Kompartimente (30, 30.1) bei Energieeintrag einen Kraftvektor (70, 70.1) entlang der optischen Achse A in Richtung der Rückseite 14 (und somit in Richtung Retina) erlauben. Die beiden Kompartimente 30.2 und 30.3 ermöglichen bei Energieeintrag einen Kraftvektor (70.2, 70.3) in entgegengesetzte Richtung. Auf diese Weise kann das Optikelement 10 gegenüber dem Haptikelement 20 entlang der optischen Achse A in beide Richtungen verschoben werden. Die axiale Linsenposition lässt sich somit im Auge anpassen, um die Sehkraft zu verbessern.

In Fig. 9 ist ein fünftes Ausführungsbeispiels für eine erfindungsgemäßen Augenlinse 1 gezeigt. Abweichend zum vierten Ausführungsbeispiel sind die Bereiche, an denen das Haptikelement 20 und die Fassungszone 18 aneinanderstoßen, als Gewinde ausgeführt. Bei den Gewinden im Haptikelement 20 und in der Fassungszone 18 handelt es sich also um zwei Führungsvorrichtungsteile. Das Kompartiment 30 ist mit dem Optikelement 10 kraftschlüssig so verbunden, dass ein Energieeintrag eine Rotation des Optikelements 10 gegenüber dem Haptikelement 20 mit dem Kraftvektor 70 (als gekrümmter Pfeil dargestellt) erlaubt. Der zugehörige Ausgangskanal 35 ist in Bezug auf den Rand des Optikelements 10 tangential orientiert, befindet sich somit in der Zeichenebene und ist deshalb nicht eingezeichnet. Ein Energieeintrag in das ebenfalls kraftschlüssig mit dem Optikelement 10 verbundene Kompartiment 30.1 erlaubt eine Rotation in entgegengesetzte Richtung (Kraftvektor 70.1). Durch eine Rotation des Optikelements 10 um die optische Achse A wird das Optikelement 10 über das Gewinde gegenüber dem Haptikelement 20 entlang der optischen Achse A verschoben. Auf diese Weise kann wie im vierten Ausführungsbeispiel die Sehkraft des Auges verbessert werden.

In Fig. 10 ist eine schematische Darstellung einer Korrekturvorrichtung 100 gezeigt. Die als Lasereinrichtung 110 ausgestaltete Energieeinrichtung emittiert einen Laserstrahl 115, der über einen Fokussiereinrichtung 120 in der in einem Auge 140 implantierten Augenlinse 1 fokussiert wird. Angesteuert wird die Lasereinheit 110 über eine Steuereinheit 130. Von dieser werden Signaldaten über eine nicht näher spezifizierte Signaldatenleitung (dargestellt als Pfeil) an die Lasereinheit 110 übertragen. Die Signaldaten werden auf Basis von Steuerdaten, die von der Planungseinheit P bereitgestellt werden, erstellt. Im dargestellten Beispiel ist die Planungseinheit P Teil Korrekturvorrichtung 100. Die Steuerdaten werden über die Schnittstelle S, die Teil der Planungseinheit P ist, über eine nicht näher spezifizierte Steuerleitung an die Steuereinheit 130 übertragen. Alternativ kann die Planungseinheit P von der Korrekturvorrichtung 100 räumlich getrennt sein.

Die Korrekturvorrichtung 100 ist weiterhin so ausgebildet, dass ihre Lage vor dem Auge 140 in allen drei Raumrichtungen beweglich positioniert werden kann (dargestellt durch drei senkrecht zueinanderstehende Doppelpfeile).

Die gezeigte Korrekturvorrichtung 100 erlaubt auf Basis der von der Planungseinrichtung P erzeugten Steuerdaten, einen Energieeintrag in der Augenlinse 1 im Auge 140 vorzunehmen, der das Haptikelement 20 und das Optikelement 10 unter Überwindung der Haftreibung gegeneinander verschiebt, und somit die Sehkraft des implantierten Auges zu verbessern.

Die vorstehend genannten und in verschiedenen Ausführungsbeispielen beschriebenen Merkmale der Erfindung sind dabei nicht nur in den angegebenen beispielhaften Kombinationen, sondern auch in anderen Kombinationen oder allein einsetzbar, ohne den Rahmen der vorliegenden Erfindung zu verlassen.

Eine auf Verfahrensmerkmale bezogene Beschreibung einer Vorrichtung gilt bezüglich dieser Merkmale analog für das entsprechende Verfahren, während Verfahrensmerkmale entsprechend funktionelle Merkmale der beschriebenen Vorrichtung darstellen.

## Patentansprüche

1. Intraokularlinse (1) mit einem Haptikelement (20) und einem Optikelement (10), wobei das Optikelement (10) mindestens zwei optische Flächen (12, 14) aufweist und relativ zum Haptikelement (20) unter Überwindung einer Haftreibung bewegt werden kann,
**dadurch gekennzeichnet, dass**
- die Intraokularlinse (1) weiterhin ein Kompartiment (30) umfasst, das mit dem Optikelement (10) oder dem Haptikelement (20) kraftschlüssig verbunden ist, wobei das Kompartiment (30) einen Ausgangskanal (35) aufweist und dazu ausgebildet ist, eine Flüssigkeit (50) aufzunehmen,
- dass die Intraokularlinse (1) weiterhin dazu ausgebildet ist, einen Energieeintrag in die ins Kompartiment (30) aufgenommene Flüssigkeit (50) zu ermöglichen, wobei der Energieeintrag in die Flüssigkeit (50) über einen Laserpuls (40) ermöglicht wird, und
- dass der Ausgangskanal (35) so ausgebildet ist, dass beim Energieeintrag in die ins Kompartiment (30) aufgenommene Flüssigkeit (50) aufgrund einer Absorption eines Teils der Energie des Energieeintrags durch die Flüssigkeit, einer daraus resultierenden expandierenden Gasblase aus Flüssigkeitsdampf und einem damit einhergehenden Druckanstieg ein Ausströmen eines Teils der Flüssigkeit (50) durch den Ausgangskanal (35) aus dem Kompartiment (30) derart erfolgt, dass eine Bewegung des Optikelements (10) als Ganzes gegenüber dem Haptikelement (20) als Ganzes unter Überwindung der Haftreibung erzeugt wird, wobei die Bewegung unter Beibehaltung der Form der mindestens zwei optischen Flächen (12, 14) erfolgt.

2. Intraokularlinse (1) nach Anspruch 1, **dadurch gekennzeichnet, dass** der Laserpuls (40) eine Pulsenergie zwischen 500µJ und 5mJ aufweist.

3. Intraokularlinse (1) nach einem der vorgenannten Ansprüche, **dadurch gekennzeichnet, dass** es sich bei der Flüssigkeit (50) um eine physiologische Salzlösung oder um Kammerwasser handelt.

4. Intraokularlinse (1) nach einem der vorgenannten Ansprüche, **dadurch gekennzeichnet, dass** die Intraokularlinse (1) weiterhin eine Führungsvorrichtung umfasst, entlang derer das Optikelement (10) gegenüber dem Haptikelement (20) unter Überwindung der Haftreibung bewegt werden kann.

5. Intraokularlinse (1) nach einem der vorgenannten Ansprüche, **dadurch gekennzeichnet, dass** das Optikelement (10) eine optische Achse (A) aufweist, und dass der Ausgangskanal (35) dazu ausgebildet ist, die Bewegung des Optikelements (10) gegenüber dem Haptikelement (20) senkrecht zur Optischen Achse (A) zu ermöglichen.

6. Intraokularlinse (1) nach einem der vorgenannten Ansprüche, **dadurch gekennzeichnet, dass** das Optikelement (10) eine optische Achse (A) aufweist, und dass der Ausgangskanal (35) dazu ausgebildet ist, die Bewegung des Optikelements (10) gegenüber dem Haptikelement (20) parallel zur optischen Achse (A) zu ermöglichen.

7. Intraokularlinse (1) nach einem der vorgenannten Ansprüche, **dadurch gekennzeichnet, dass** das Optikelement (10) eine optische Achse (A) aufweist, und dass der Ausgangskanal (35) dazu ausgebildet ist, die Bewegung des Optikelements (10) gegenüber dem Haptikelement (20) rotierend um die optische Achse (A) zu ermöglichen.

8. Intraokularlinse (1) nach einem der vorgenannten Ansprüche, **dadurch gekennzeichnet, dass** das Haptikelement (20) einen Optikbereich 21 aufweist, der mindestens zwei optisch wirksame Flächen (22, 24) umfasst.

9. Intraokularlinse (1) nach einem der vorgenannten Ansprüche, **dadurch gekennzeichnet, dass** die Intraokularlinse (1) mindestens ein weiteres Kompartiment (30.1, 30.2, 30.3) umfasst, das mit dem Optikelement (10) kraftschlüssig verbunden ist, wobei der Ausgangskanal (35) des mindestens einen weiteren Kompartiments (30.1, 30.2, 30.3) so ausgebildet ist, dass beim Energieeintrag in die ins mindestens eine weitere Kompartiment (30.1, 30.2, 30.3) aufgenommene Flüssigkeit (50) aufgrund einer Absorption eines Teils der Energie des Energieeintrags durch die Flüssigkeit, einer daraus resultierenden expandierenden Gasblase aus Flüssigkeitsdampf und einem damit einhergehenden Druckanstieg ein Ausströmen eines Teils der Flüssigkeit (50) durch den Ausgangskanal (35) aus dem mindestens einen weiteren Kompartiment (30.1, 30.2, 30.3) derart erfolgt, dass eine Bewegung des Optikelements (10) gegenüber dem Haptikelement (20) unter Überwindung der Haftreibung mit einer Bewegungsrichtung erzeugt wird, die verschieden ist von einer Bewegungsrichtung, die durch ein Ausströmen der Flüssigkeit (50) aus dem ersten Kompartiment (30) erzeugt wird.

10. Planungseinheit (P) zur Erzeugung von Steuerdaten für eine Korrekturvorrichtung (100) zur Korrektur eines Refraktionsfehlers eines Auges (140), das eine Intraokularlinse (1) nach einem der Ansprüche 1 bis 9 aufweist, wobei die Korrekturvorrichtung (100)
- eine Energieeinrichtung zur Bereitstellung einer Energie,
- eine Fokussiereinrichtung (120) zum Fokussieren der Energie in einem Energiefokus, und
- eine Steuereinheit (130) zu Steuerung der Korrekturvorrichtung (100) mittels der Steuerdaten
umfasst,
wobei die Planungseinheit (P) eine Schnittstelle (S) zum Abführen der Steuerdaten an die Steuereinheit (130) aufweist,
**dadurch gekennzeichnet, dass** die Planungseinheit (P) dazu ausgebildet ist, weitere Steuerdaten zu erzeugen, mit denen die Korrekturvorrichtung (100) so angesteuert werden kann, dass die Energieeinrichtung (110) die Energie bereitstellt, die im Kompartiment (30) der Intraokularlinse (1) den Energieeintrag ermöglicht, der die Bewegung des Optikelements (10) gegenüber dem Haptikelement (20) unter Überwindung der Haftreibung erzeugt.

11. Korrekturvorrichtung (100) zur Korrektur eines Refraktionsfehlers eines Auges (140), das eine Intraokularlinse (1) nach einem der Ansprüche 1 bis 9 aufweist, umfassend:
- eine Energieeinrichtung (110) zur Bereitstellung einer Energie,
- eine Fokussiereinrichtung (120) zum Fokussieren der Energie in einem Energiefokus,
- eine Steuereinheit (130) zu Steuerung der Korrekturvorrichtung (100) mittels Steuerdaten, sowie
- eine Planungseinheit (P) zur Erzeugung der Steuerdaten nach Anspruch 11.

12. Planungsverfahren zur Erzeugung von Steuerdaten für eine Korrekturvorrichtung (100) zur Korrektur eines Refraktionsfehlers eines Auges (140), das eine Intraokularlinse (1) nach einem der Ansprüche 1 bis 9 aufweist, wobei die Korrekturvorrichtung (100)
- eine Energieeinrichtung (110) zur Bereitstellung einer Energie,
- eine Fokussiereinrichtung (120) zum Fokussieren der Energie in einem Energiefokus, und
- eine Steuereinheit (130) zu Steuerung der Korrekturvorrichtung (100) mittels der Steuerdaten
umfasst,
**dadurch gekennzeichnet, dass** das Planungsverfahren folgende Schritte umfasst:
- Erzeugung weitere Steuerdaten, mit denen die Korrekturvorrichtung (100) so angesteuert werden kann, dass die Energieeinrichtung (110) eine Energie bereitstellt, die im Kompartiment (30) der Intraokularlinse (1) den Energieeintrag ermöglicht, der die Bewegung des Optikelements (10) gegenüber dem Haptikelement (20) unter Überwindung der Haftreibung erzeugt, und
- Zuführung der Steuerdaten zu der Steuereinheit (130) der Korrekturvorrichtung (100).

## Claims

1. Intraocular lens (1) having a haptic element (20) and an optical element (10), wherein the optical element (10) comprises at least two optical surfaces (12, 14) and can be moved relative to the haptic element (20) upon overcoming static friction,
**characterized in that**
- the intraocular lens (1) furthermore comprises a compartment (30) that is frictionally connected to the optical element (10) or to the haptic element (20), wherein the compartment (30) comprises an outlet channel (35) and is designed to accommodate a fluid (50),
- **in that** the intraocular lens (1) is furthermore configured to enable an input of energy into the fluid (50) accommodated in the compartment (30), the input of energy into the fluid (50) being made possible by means of a laser pulse (40), and
- **in that** the outlet channel (35) is configured such that, when energy is introduced into the fluid (50) accommodated in the compartment (30) on account of the fluid absorbing a portion of the energy of the energy input, a resulting expanding gas bubble comprising liquid vapour and an associated pressure increase is accompanied by an outflow of a portion of the fluid (50) from the compartment (30) through the outlet channel (35), in such a way that a movement of the optical element (10) as a whole vis-à-vis the haptic element (20) as a whole is generated upon overcoming the static friction, wherein the movement takes place while retaining the shape of the at least two optical surfaces (12, 14).

2. Intraocular lens (1) according to Claim 1, **characterized in that** the laser pulse (40) has a pulse energy between 500 µJ and 5 mJ.

3. Intraocular lens (1) according to either of the preceding claims, **characterized in that** the fluid (50) is normal saline or aqueous humour.

4. Intraocular lens (1) according to any of the preceding claims, **characterized in that** the intraocular lens (1) furthermore comprises a guide device along which the optical element (10) can be moved vis-à-vis the haptic element (20) upon overcoming the static friction.

5. Intraocular lens (1) according to any of the preceding claims, **characterized in that** the optical element (10) has an optical axis (A), and **in that** the outlet channel (35) is configured to enable the movement of the optical element (10) vis-à-vis the haptic element (20) in a manner perpendicular to the optical axis (A).

6. Intraocular lens (1) according to any of the preceding claims, **characterized in that** the optical element (10) has an optical axis (A), and **in that** the outlet channel (35) is configured to enable the movement of the optical element (10) vis-à-vis the haptic element (20) in a manner parallel to the optical axis (A).

7. Intraocular lens (1) according to any of the preceding claims, **characterized in that** the optical element (10) has an optical axis (A), and **in that** the outlet channel (35) is configured to enable the movement of the optical element (10) vis-à-vis the haptic element (20) in a manner rotating about the optical axis (A).

8. Intraocular lens (1) according to any of the preceding claims, **characterized in that** the haptic element (20) has an optical region 21 that comprises at least two optically effective surfaces (22, 24).

9. Intraocular lens (1) according to any of the preceding claims, **characterized in that** the intraocular lens (1) comprises at least one further compartment (30.1, 30.2, 30.3) that is frictionally connected to the optical element (10), wherein the outlet channel (35) of the at least one further compartment (30.1, 30.2, 30.3) is configured such that, when energy is introduced into the fluid (50) accommodated in the at least one further compartment (30.1, 30.2, 30.3) on account of the fluid absorbing a portion of the energy of the energy input, a resulting expanding gas bubble comprising liquid vapour and an associated pressure increase is accompanied by an outflow of a portion of the fluid (50) from the at least one further compartment (30.1, 30.2, 30.3) through the outlet channel (35), in such a way that a movement of the optical element (10) vis-à-vis the haptic element (20) is generated with a movement direction upon overcoming the static friction, wherein the movement direction differs from a movement direction generated by an outflow of the fluid (50) from the first compartment (30).

10. Planning unit (P) for generating control data for a correction device (100) for correcting a refractive error of an eye (140) comprising an intraocular lens (1) according to any of Claims 1 to 9, wherein the correction device (100) comprises
- an energy apparatus for providing energy,
- a focusing apparatus (120) for focusing the energy in an energy focus and
- a control unit (130) for controlling the correction device (100) by means of the control data,
wherein the planning unit (P) comprises an interface (S) for transferring the control data to the control unit (130),
**characterized in that** the planning unit (P) is configured to generate further control data with which the correction device (100) can be controlled such that the energy apparatus (110) provides the energy which enables the energy input in the compartment (30) of the intraocular lens (1) that generates the movement of the optical element (10) vis-à-vis the haptic element (20) upon overcoming the static friction.

11. Correction device (100) for correcting a refractive error of an eye (140) comprising an intraocular lens (1) according to any of Claims 1 to 9, comprising:
- an energy apparatus (110) for providing energy,
- a focusing apparatus (120) for focusing the energy in an energy focus,
- a control unit (130) for controlling the correction device (100) by means of control data and
- a planning unit (P) for generating the control data according to Claim 11.

12. Planning method for generating control data for a correction device (100) for correcting a refractive error of an eye (140) comprising an intraocular lens (1) according to any of Claims 1 to 9, wherein the correction device (100) comprises
- an energy apparatus (110) for providing energy,
- a focusing apparatus (120) for focusing the energy in an energy focus and
- a control unit (130) for controlling the correction device (100) by means of the control data,
**characterized in that** the planning method comprises the following steps:
- generating further control data with which the correction device (100) can be controlled such that the energy apparatus (110) provides an energy which enables the energy input in the compartment (30) of the intraocular lens (1) that generates the movement of the optical element (10) vis-à-vis the haptic element (20) upon overcoming the static friction and
- supplying the control data to the control unit (130) of the correction device (100).

## Revendications

1. Lentille intraoculaire (1) avec un élément haptique (20) et un élément optique (10), l'élément optique (10) comportant au moins deux surfaces optiques (12, 14) et pouvant être déplacé par rapport à l'élément haptique (20) en surmontant un frottement statique,
**caractérisée en ce que**
- la lentille intraoculaire (1) comprend par ailleurs un compartiment (30), qui est relié à force à l'élément optique (10) ou à l'élément haptique (20), le compartiment (30) comportant un canal de sortie (35) et étant formé pour recevoir un fluide (50),
- que la lentille intraoculaire (1) est par ailleurs formée pour permettre un apport d'énergie dans le fluide (50) reçu dans le compartiment (30), l'énergie apportée dans le fluide (50) étant rendue possible par une impulsion laser (40), et
- que le canal de sortie (35) est formé de telle sorte que, qu'en cas d'apport d'énergie dans le fluide (50) reçu dans le compartiment (30), en raison d'une absorption d'une partie de l'énergie de l'énergie apportée par le fluide , d'une bulle de gaz en expansion en résultant composée de vapeur de fluide et d'une augmentation de pression concomitante, une partie du fluide (50) s'écoule hors du compartiment (30) par le canal de sortie (35) de telle manière qu'un mouvement de l'élément optique (10) dans son ensemble est généré par rapport à l'élément haptique (20) dans son ensemble en surmontant le frottement statique, le mouvement s'effectuant tout en maintenant la forme des au moins deux surfaces optiques (12, 14).

2. Lentille intraoculaire (1) selon la revendication 1, **caractérisée en ce que** l'impulsion laser (40) présente une énergie d'impulsion entre 500 µJ et 5 mJ.

3. Lentille intraoculaire (1) selon l'une des revendications précédentes, **caractérisée en ce que** le fluide (50) est une solution saline physiologique ou une humeur aqueuse.

4. Lentille intraoculaire (1) selon l'une des revendications précédentes, **caractérisée en ce que** la lentille intraoculaire (1) comprend par ailleurs un dispositif de guidage, le long duquel l'élément optique (10) peut être déplacé par rapport à l'élément haptique (20) tout en surmontant le frottement statique.

5. Lentille intraoculaire (1) selon l'une des revendications précédentes, **caractérisée en ce que** l'élément optique (10) comporte un axe optique (A), et que le canal de sortie (35) est formé pour permettre le déplacement de l'élément optique (10) par rapport à l'élément haptique (20) perpendiculairement à l'axe optique (A).

6. Lentille intraoculaire (1) selon l'une des revendications précédentes, **caractérisée en ce que** l'élément optique (10) comporte un axe optique (A), et que le canal de sortie (35) est formé pour permettre le déplacement de l'élément optique (10) par rapport à l'élément haptique (20) parallèlement à l'axe optique (A).

7. Lentille intraoculaire (1) selon l'une des revendications précédentes, **caractérisée en ce que** l'élément optique (10) comporte un axe optique (A), et que le canal de sortie (35) est formé pour permettre le déplacement en rotation de l'élément optique (10) par rapport à l'élément haptique (20) autour de l'axe optique (A).

8. Lentille intraoculaire (1) selon l'une des revendications précédentes, **caractérisée en ce que** l'élément haptique (20) comporte une zone optique (21), qui comprend au moins deux surfaces optiquement actives (22, 24).

9. Lentille intraoculaire (1) selon l'une des revendications précédentes, **caractérisée en ce que** la lentille intraoculaire (1) comprend au moins un autre compartiment (30.1, 30.2, 30.3), qui est relié à force à l'élément optique (10), le canal de sortie (35) de l'au moins un autre compartiment (30.1, 30.2, 30.3) étant formé de telle sorte qu'en cas d'apport d'énergie dans le fluide (50) reçu dans l'au moins un autre compartiment (30.1, 30.2, 30.3) en raison d'une absorption d'une partie de l'énergie de l'énergie apportée par le fluide , d'une bulle de gaz en expansion en résultant composée de vapeur de liquide et d'une augmentation de pression concomitante, une partie du fluide (50) s'écoule hors de l'au moins un autre compartiment (30.1, 30.2, 30.3) à travers le canal de sortie (35) de telle manière qu'un mouvement de l'élément optique (10) par rapport à l'élément haptique (20) est généré en surmontant le frottement statique avec une direction de mouvement, qui est différente d'une direction de mouvement qui est générée par un écoulement du fluide (50) hors du premier compartiment (30).

10. Unité de planification (P) pour générer des données de commande pour un dispositif de correction (100) pour corriger un défaut de réfraction d'un œil (140), qui comporte une lentille intraoculaire (1) selon l'une des revendications 1 à 9, le dispositif de correction (100) comprenant
- un système d'énergie pour la fourniture d'énergie,
- un système de focalisation (120) pour focaliser l'énergie dans un foyer énergétique, et
- une unité de commande (130) pour commander le dispositif de correction (100) au moyen des données de commande,
l'unité de planification (P) comportant une interface (S) pour transférer les données de commande à l'unité de commande (130),
**caractérisée en ce que** l'unité de planification (P) est formée pour générer d'autres données de commande, avec lesquelles le dispositif de correction (100) peut être piloté de telle sorte que le système d'énergie (110) fournit l'énergie qui permet l'apport d'énergie dans le compartiment (30) de la lentille intraoculaire (1), qui génère le mouvement de l'élément optique (10) par rapport à l'élément haptique (20) en surmontant le frottement statique.

11. Dispositif de correction (100) pour corriger un défaut de réfraction d'un œil (140), qui comporte une lentille intraoculaire (1) selon l'une des revendications 1 à 9, comprenant :
- un système d'énergie (110) pour la fourniture d'énergie,
- un système de focalisation (120) pour focaliser l'énergie dans un foyer énergétique,
- une unité de commande (130) pour commander le dispositif de correction (100) au moyen de données de commande, et
- une unité de planification (P) pour générer les données de commande selon la revendication 11.

12. Procédé de planification pour générer des données de commande pour un dispositif de correction (100) pour corriger un défaut de réfraction d'un œil (140), qui comporte une lentille intraoculaire (1) selon l'une des revendications 1 à 9, le dispositif de correction (100) comprenant
- un système d'énergie (110) pour la fourniture d'énergie,
- un système de focalisation (120) pour focaliser l'énergie dans un foyer énergétique, et
- une unité de commande (130) pour commander le dispositif de correction (100) au moyen des données de commande,
**caractérisé en ce que** le procédé de planification comprend des étapes suivantes :
- génération d'autres données de commande, avec lesquelles le dispositif de correction (100) peut être piloté de telle sorte que le système d'énergie (110) fournit une énergie, qui permet l'apport d'énergie dans le compartiment (30) de la lentille intraoculaire (1, qui génère le mouvement de l'élément optique (10) par rapport à l'élément haptique (20) en surmontant le frottement statique, et
- amenée des données de commande à l'unité de commande (130) du dispositif de correction (100).
